(19)

Europäisches Patentamt
European Patent Office
Office européen des brevets

(11) **EP 3 305 367 A1**

(12) **EUROPEAN PATENT APPLICATION**

(43) Date of publication:
**11.04.2018 Bulletin 2018/15**

(21) Application number: **16192790.0**

(22) Date of filing: **07.10.2016**

(51) Int Cl.:
**A61N 5/10** (2006.01)     **A61B 6/03** (2006.01)
**A61B 6/00** (2006.01)     **A61B 5/055** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **ION BEAM APPLICATIONS S.A.**
**1348 Louvain-la-Neuve (BE)**

(72) Inventor: **PRIEELS, Damien**
**1348 Louvain-la-Neuve (BE)**

(74) Representative: **Pecher, Nicolas et al**
**Pecher & Partners**
**Rue Louis de Geer, 6**
**1348 Louvain-la-Neuve (BE)**

(54) **MEDICAL APPARATUS COMPRISING A HADRON THERAPY DEVICE, A MRI, AND A HADRON RADIOGRAPHY SYSTEM**

(57)     A medical apparatus that comprises:
(A) a hadron therapy device adapted for directing an imaging hadron beam having an initial imaging beam energy, along a beam path, said beam path going through and beyond:
• a subject of interest comprising a plurality of tissues and crossed by the beam path between upstream and downstream boundaries, and
• a target spot located inside a target tissue of the subject of interest;

(B) a magnetic resonance imaging device for acquiring magnetic resonance data within an imaging volume comprising a portion of the subject of interest including the target spot and at least the portion of the beam path comprised between the upstream boundary and the target spot;
(C) a hadron radiography system (HRS) adapted for acquiring a signal generated by the imaging hadron beam; and
(D) a controller configured for determining a water equivalent path length of the beam path between upstream and downstream boundaries, said determination being based, at least, on the signal acquired with the HRS.

Figure 7

EP 3 305 367 A1

**Description**

**Field of the invention**

According to a first aspect, the invention relates to a medical apparatus comprising a charged hadron therapy device, a magnetic resonance imaging device, and a hadron radiography system. According to a second aspect, the invention relates to methods for establishing and/or checking a treatment plan.

**Description of prior art**

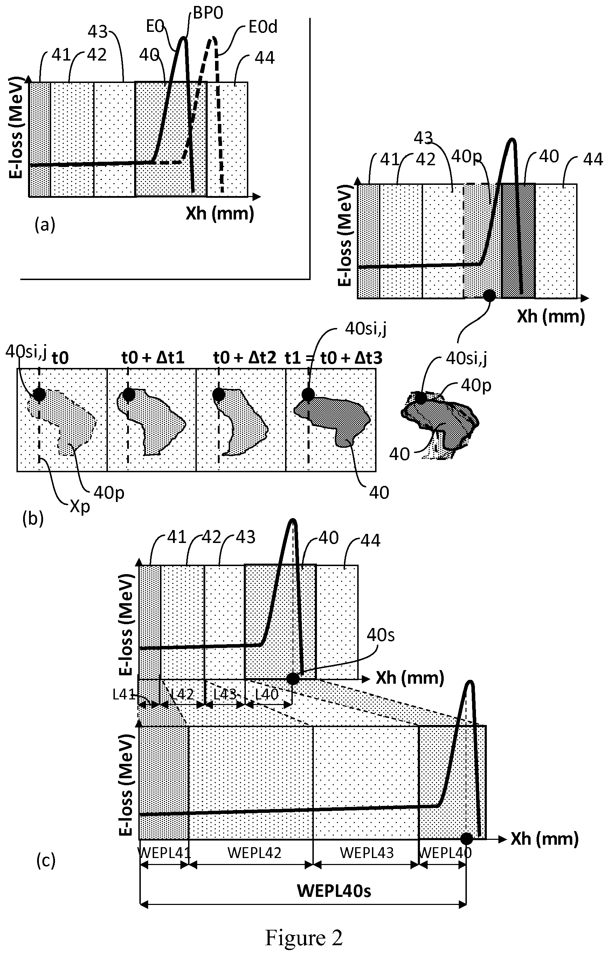

Hadron therapy (for example, proton therapy) for treating a patient has been known for a couple of decades with the prospect of several advantages over conventional radiotherapy. These advantages are due to the physical nature of hadrons. A photon beam in conventional radiotherapy releases its energy according to a decreasing exponential curve as a function of the distance of tissue traversed by the photon beam. By contrast and such as illustrated in Figure 2, a hadron beam first releases a small fraction of its energy as it penetrates tissues **41-43,** forming a plateau then, as the hadron path is prolonged, the whole energy is released locally following a steep increase to a peak and a fall-off at the end of the range of the beam. The peak is called Bragg peak and corresponds to the maximum of the Bragg curve illustrated in Figure 2(c). Consequently, a hadron beam can deliver a high dose of hadrons at a precise location within a target tissue **40,** thus preserving the surrounding healthy tissues **41-44.** As illustrated in Figure 2(a), the advantage of hadron therapy allowing the delivery of high doses of hadron at a precise location is also one of its weaknesses, because if the position, **BP0,** of the Bragg peak of a hadron beam is offset relative to the target tissues **40,** high doses of hadrons may be delivered to adjacent tissues **43, 44** which are healthy (compare solid line, **E0,** and dashed line, **E0d,** of the curves of energy loss, $E_{loss}$, with respect to the distance, **Xh,** travelled by the hadron beam within tissues and measured along the beam path, **Xp,** in Figure 2(a)). For this reason, the determination of the relative position of the Bragg peak with respect to the position of the target tissue is crucial to properly implement hadron therapy to a patient.

In practice, hadron therapy usually requires the establishment of a treatment plan before any treatment can start. During this treatment plan, a computer tomography scan (CT scan) of the patient and target tissues is usually performed. The CT scan is used to characterize the target tissue **40** and the surrounding tissues **41-43** to be traversed by a treatment hadron beam **1h** for the treatment of a patient. The characterization yields a 3D representation of the volume comprising the target tissue, and a treatment plan system determines a range-dose calculated based on the nature of the tissues **41-43** traversed by the hadron beam.

This characterization permits computation of a water equivalent path length (WEPL), which is used for determining the initial energy, **Ek,** of the treatment hadron beam required for delivering a prescribed dose of hadrons to a target spot **40s,** wherein k = 0 or 1 depending on the stage when said initial energy was determined. Figure 2(c) illustrates the conversion of the physical distances travelled by a hadron beam traversing different tissues into corresponding WEPL's. The WEPL of a hadron beam travelling a given distance through a given tissue is the equivalent distance said hadron beam would travel in water. As illustrated in Figure 2(c) if, as is usually the case, healthy tissues **41-43** of different natures and thicknesses separate a target tissue from the outer surface of the skin of a patient, the WEPL of a target spot is calculated taking into account the water corresponding path lengths of each tissue in series until the target spot is reached. With a value of the equivalent path length of a hadron beam traveling in water, the initial energy, **Ek,** required for positioning the Bragg peak at the WEPL of the target spot can easily be computed and corresponds to the initial energy, **Ek,** required for positioning the Bragg peak at the target spot within the target tissue.

The treatment plan can then be executed during a treatment phase including one or more treatment sessions during which doses of hadrons are deposited onto the target tissue. The position of the Bragg peak of a hadron beam with respect to the target spots of a target tissue, however, suffers of a number of uncertainties including:

- the variations of the patient position, on the one hand, during a hadron therapy session and, on the other hand, between the establishment of the treatment plan and the hadron therapy session;

- the variations of the size and/or of the position of the target tissue (see Figure 2(b)) and/or of the healthy tissues **41-43** positioned upstream from the target tissue with respect to the hadron beam.

- the range calculation from CT scans is limited by the quality of the CT images. Another limitation is linked to the fact that CT scans use the attenuation of X-rays that have to be converted in hadron attenuation which is non obvious and depends on the chemical composition of the tissues traversed.

The uncertainty on the position of the patient and, in particular, of the target tissue is critical for obvious reasons. Even with an accurate characterization by CT scan, the actual position of a target tissue during a treatment session remains difficult to ascertain for the following reasons:

> (A) first, during an irradiation session, the position of a target tissue can change because of anatomical processes such as breathing, digestion, or heartbeats of the patient. Anatomical processes can also cause gases or fluids appearing or disappearing from the beam path, **Xp,** of a hadron beam.

> (B) second, treatment plans are usually determined several days or weeks before a hadron treatment session starts and treatment of a patient can take several weeks distributed over several treatment sessions. During this time period, the patient can lose or gain weight, therefore modifying, sometimes significantly, the volume of tissues such as fats and muscles.

Accordingly, the size of the target tissue can change (e.g. a tumour may have grown, receded, or changed position or geometry). Figure 2(b) shows an example of evolution of the size and position of a target tissue **40** between the time, **t0,** of the establishment of the treatment plan and the times, **t0 + ∆t1, t0 + ∆t2, t1 = t0 + ∆t3,** of treatment sessions. The treatment plan and last treatment session may be separated by several days or weeks. The treatment plan established at time, **t0,** may therefore comprise irradiation of a target spot **40si,j** (black spot in Figure 2(b)) which belonged to the target tissue **40p** at said time, **t0.** Because the target tissue **40p** may have moved or changed shape during the period, ∆**t3,** said target spot **40si,j** may not belong to the target tissue **40** anymore at the time, **t0 + ∆t3,** of the treatment session and be located in a healthy tissue instead. Consequently, irradiating said target spot would hit and possibly harm healthy tissues **43** instead of target tissues **40.**

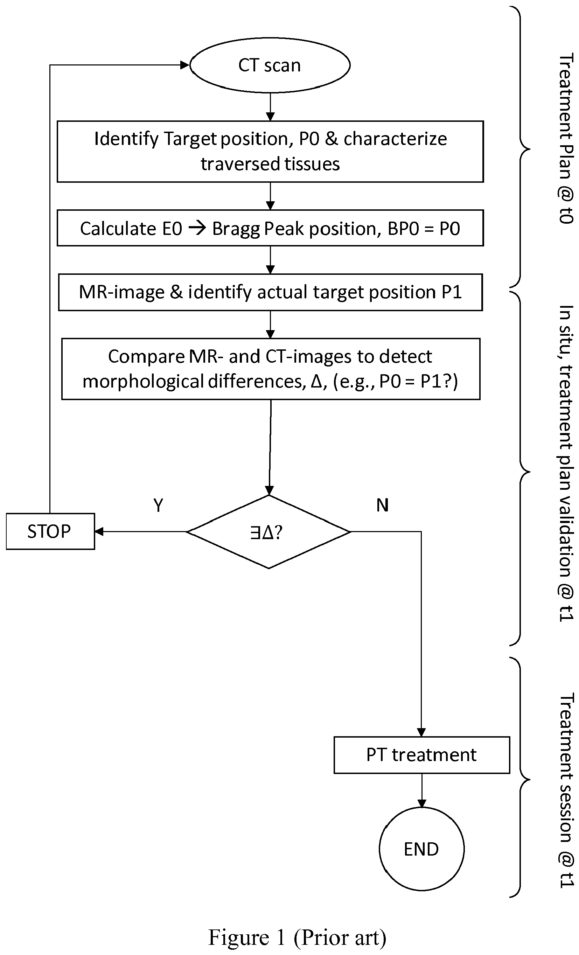

The use of a magnetic resonance imaging device (MRI) coupled to a hadron therapy device has been proposed in the art for identifying any variation of the size and /or the position of a target tissue. For example, US patent 8427148 describes a system comprising a hadron therapy device coupled to a MRI. Said system can acquire images of the patient during a hadron therapy session and can compare these images with CT scan images of the treatment plan. Present Figure 1 illustrates an example of a known flowchart of a hadron therapy session using a hadron therapy device coupled to an MRI. A treatment plan is established including the characterization of the target tissue 40s and surrounding tissues **41-43.** This step is traditionally performed with a CT scan analysis and allows the determination of the position, **P0,** and morphology of a target tissue, the best trajectories or beam paths, **Xp,** of hadron beams for the hadron treatment of the target tissue, and characterization of the sizes and natures of the tissues traversed by a hadron beam following said beam paths, **Xp,** to determine **WEPL40s's** of target spots of the said target tissue. The initial energies, **Ek,** of the hadron beams required for matching the corresponding positions, **BP0,** of the Bragg peaks of the hadron beams to the position, **P0,** of the target tissue can thus be calculated. This completes the establishment of a treatment plan.

A hadron therapy session follows the establishment of the treatment plan. With an MRI coupled to a hadron therapy device, it is possible to capture a magnetic resonance (MR) image of a volume, **Vp,** including the target tissue and surrounding tissues to be traversed by a hadron beam. The MR image can then be compared with CT scan images to assess whether any morphological differences, ∆, can be detected in the imaged tissues between the time the CT scans were performed (= **t0** in Figure 2(b)) and the time of the hadron therapy session **(t1= t0 + ∆ t3** in Figure 2(b)). If no substantial difference in morphology affecting the treatment session can be detected, then the hadron therapy session proceeds as planned in the treatment plan. If, on the other hand, some differences are detected that could influence the relative position of the target tissue with respect to the planned hadron beams and their respective Bragg peaks, the hadron therapy session is interrupted and a new treatment plan must be established. This technique proposed in the art is greatly advantageous because it can prevent carrying out a hadron therapy session based on a treatment plan which has become obsolete, thus preventing healthy tissues from being irradiated instead of the target tissue.

The magnetic resonance (MR) images provide high contrast of soft tissue traversed by a hadron beam but, to date, have not been suitable for visualizing the hadron beam itself, let alone the position of the Bragg peak because:

• MRI measures the density of hydrogen atoms in tissues, but to date does not yield any identifiable information on the hadron stopping power ratio. The conversion from density of hydrogen atoms to the hadron stopping power ratio suffers from uncertainties similar to and yet less understood than those of the conversion from X-rays in CT scan.

• Due to the different techniques used in CT scan and in MRI, the comparison between the images from CT scan and the images from MRI is not obvious and can suffer from uncertainties.

In conclusion, whilst in hadron therapy, an accurate determination of the position of the Bragg peak relative to the portion of a target tissue is crucial because errors on this position may lead to the irradiation of healthy tissues rather than irradiation of target tissues, no satisfactory solution for determining the relative positions of the Bragg peak and target tissues is available to date. Apparatuses combining a hadron therapy device and a MRI proposed in the art *allow in situ* acquisition of images during a treatment session thus giving information related to the actual position of the target tissue. Said images are, however, not sufficient for ensuring the exact determination of the position of the Bragg peak of a hadron beam and of where it stands relative to the target tissue. There therefore remains a need for a hadron therapy device combined to MRI allowing a better determination of the position of the Bragg peak relative to the position of a target tissue.

**Summary of the invention**

The present invention is defined in the appended independent claims. Preferred embodiments are defined in the dependent claims.

According to a first aspect, the invention relates to a medical apparatus comprising:

(A) a hadron therapy device comprising a hadron source adapted for directing an imaging hadron beam having an initial imaging beam energy, E0p, along a beam path, said beam path going through and beyond:

- a subject of interest comprising a plurality of tissues m and crossed by the beam path between upstream and downstream boundaries, and

- a target spot located inside a target tissue of the subject of interest between the upstream and downstream boundaries;

(B) a magnetic resonance imaging device (MRI) for acquiring magnetic resonance (MR) data within an imaging volume, Vp, comprising a portion of the subject of interest including the target spot and at least the portion of the beam path comprised between the upstream boundary and the target spot;

(C) a hadron radiography system (HRS) adapted for acquiring a signal generated by the imaging hadron beam; and

(D) a controller configured for a determination of a water equivalent path length WEPL,HRS of the beam path between said upstream boundary and said downstream boundary, said determination being based, at least, on the signal acquired with the HRS.

The controller can be further configured for computing the water equivalent path length, WEPLm, of the plurality tissues m, of thickness Lm, crossed by the beam path comprised between the upstream and downstream boundaries, the computation being based on the MR data and on the WEPL,HRS.

Preferably, the controller is configured for performing, at a time t1 during a treatment session, a validation of a planned value of a water equivalent path length, WEPL40s,0, of a beam path comprised between the upstream boundary and the target spot (40s), wherein said planned value of the WEPL40s,0 had been determined previously at time t0 during a treatment session. The validation may comprise:

- a calculation of an actual value of a water equivalent path length WEPL40s,1 of a beam path comprised between the upstream boundary and the target spot, said calculation being based on the WEPLm's of the plurality of tissues positioned upstream of and including the target tissue;

- a comparison of the actual value of the WEPL40s,1 with the planned value of the WEPL40s,0;

- if $WEPL40s,0 - \delta \leq WEPL40s,1 \leq WEPL40s,0 + \delta$ with $\delta = 10$ mm, preferably $\delta = 5$ mm, more preferably $\delta = 3$ mm, then apply the treatment plan;

- if $WEPL40s,1 \geq WEPL40s,0 + \delta$ or $WEPL40s,1 \leq WEPL40s,0 - \delta$ with $\delta = 10$ mm, preferably $\delta = 5$ mm, more preferably $\delta = 3$ mm, then take further actions.

Preferably, the further actions comprise a correction of:

- a planned initial energy, E0, of a treatment hadron beam previously computed during a treatment plan and based on the planned value of the WEPL40s,0, said planned initial energy, E0, yielding the Bragg peak at a planned position, BP0, corresponding to the position of the target spot as defined by treatment plan; to

- a corrected initial energy, E1, of a treatment hadron beam computed during the validation and based on the actual value of the WEPL40s,1, said corrected initial energy, E1, yielding the Bragg peak at a treatment position, BP1,

corresponding to the actual position of the target spot.

The controller may also be configured for establishing a treatment plan by:

- calculating a preliminary value of WEPL40s,0 of the target spot said calculation being based on the WEPLm's; and

- computing a planned initial energy, E0, of a treatment hadron beam, the computation being based on the value of WEPL40s,0 and yielding the Bragg peak BP0, at a planned position corresponding to the position of the target spot during the establishment of the treatment plan.

The hadron radiography system of the medical apparatus according to the invention can comprise one or more of the following detectors: a range telescope, a calorimeter, or a spectrometer.

In a prefered embodiment, the medical apparatus according to the present invention, further comprises at least one of a prompt-gamma detector and a PET scan.

In a prefered embodiment, the medical apparatus according to the present invention, further comprises a support for supporting a patient in a non-supine position.

According to a second aspect, the invention relates to a method for validating a planned initial energy, E0, of a treatment hadron beam computed during a treatment plan for irradiation of a target spot inside a subject of interest, said subject of interest comprising a plurality of tissues m. The method comprises the following steps:

(A) performing a magnetic resonance (MR) imaging of an imaging volume, Vp, comprising the target spot, and acquiring MR data;

(B) emitting, along a beam path, an imaging hadron beam having an initial imaging beam energy, E0p, said beam path going through and beyond:

- the subject of interest extending along the beam path between upstream and downstream boundaries, and

- the target spot;

(C) detecting a signal generated by said imaging hadron beam with a hadron radiography system (HRS);

(D) determining, from said signal, the water equivalent path length WEPL,HRS of said beam path between upstream and downstream boundaries;

(E) computing the WEPLm of tissues m, of thickness Lm, crossed by the beam path comprised between the upstream and downstream boundaries, the computation being based on the MR data and on the WEPL,HRS.

(F) computing the value of an actual value of the WEPL40s,1 of the beam path comprised between the upstream boundary and the target spot, said calculation being based on the WEPLm's of the tissues positioned upstream of and including the target spot;

(G) comparing the actual value of the WEPL40s,1 with a preliminary value of WEPL40s,0 determined during the establishment of the treatment plan;

(H) if WEPL40s,0 - tol $\leq$ WEPL40s,1 $\leq$ WEPL40s,0 + tol with tol = 10 mm, preferably tol = 5 mm, more preferably tol = 3 mm, validating the planned initial energy, E0;

(I) if WEPL40s,1 $\geq$ WEPL40s,0 + tol or WEPL40s,1 $\leq$ WEPL40s,0 - tol with tol = 10 mm, preferably tol = 5 mm, more preferably tol = 3 mm, taking further actions.

Prefereably, the further actions comprise a modification of the value of the planned initial energy, E0 to a value of a corrected initial energy, E1, of a treatment hadron beam computed during the validation and based on the WEPL40s, 1, said corrected initial energy, E1, yielding the Bragg peak at a treatment position, BP1, corresponding to the actual position of the target spot.

The validation of the treatment plan may be done at a time t0 + $\Delta$ti and may be preceded by an establishment of the treatment plan at a time t0. The establishment of the treatment plan comprises the following steps:

(A) running a CT scanner and / or a magnetic resonance imaging to yield a preliminary characterisation of the tissues within the portion of the subject of interest including a predicted beam path comprised between upstream boundary and target spot, and an identification of a preliminary WEPL40s,0 of the beam path comprised between the upstream boundary and the target spot; and,

(B) computing a dose delivery scheme optimized with respect to the preliminary characterization, the computation including the computation of a planned initial energy, E0, of a treatment hadron beam, the computation being based on the value of WEPL40s,0 and yielding the Bragg peak at a planned position, BP0, corresponding to the position of the target spot during the establishment of the treatment plan.

Preferably, the method of establishment of a treatment plan of a treatment hadron beam for irradiation of a target spot inside a subject of interest comprises the following steps:

(A) performing a magnetic resonance (MR) imaging of an imaging volume, Vp, comprising a target spot for acquiring MR data;

(B) emitting, along a beam path, an imaging hadron beam having an initial imaging beam energy, E0p, said beam path going through and beyond:

- the subject of interest extending along the beam path between upstream and downstream boundaries, and

- the target spot;

(C) detecting a signal generated by said imaging hadron beam with a hadron radiography system (HRS);

(D) determining, from said signal, the water equivalent path length WEPL,HRS of said beam path between upstream and downstream boundaries;

(E) computing the WEPLm's of tissues m, of thickness Lm, crossed by the beam path comprised between the upstream and downstream boundaries, the computation being based on the MR data and on the WEPL,HRS.

(F) computing of a value of the WEPL40s,0 of the beam path comprised between the upstream boundary and the target spot, said calculation being based on the WEPLm's of the tissues positioned upstream of and including the target spot;

(G) computing a dose delivery scheme including the computation of a planned initial energy, E0, of a treatment hadron beam, the computation being based on the value of WEPL40s,0 and yielding the Bragg peak at a planned position, BP0, corresponding to the position of the target spot during the establishment of the treatment plan.

Preferably, the method of establishment of a treatment plan further comprises a preliminary characterisation, of the tissues crossed by the beam path within the portion of the subject of interest including the beam path comprised between upstream and downstream boundaries. The characterisation may be based, at least, on the MR data.

Preferably, the validation of the treatment plan is done at a time t1 according to the method of the present invention and is preceded by an establishment of the treatment plan at a time t0 according to the method of the present invention.

Preferably, the steps of performing a magnetic resonance (MR) imaging and of emitting an imaging hadron beam are done in the same room.

Preferably, the methods according to the present invention, further comprise the step of providing a medical apparatus according to the present invention.

## Short description of the drawings

These and further aspects of the invention will be explained in greater detail by way of example and with reference to the accompanying drawings in which:

**Figure 1** shows a flowchart of a state of the art hadron therapy method using a hadron therapy device coupled to a MRI;

**Figure 2** schematically shows (a) the position of the Bragg peak of a hadron beam traversing tissues, (b) that changes with time of the morphology and position of a target tissue can create a discrepancy between a treatment plan and an actual required treatment, and (c) relationship between actual path lengths and water equivalent path lengths.

**Figure 3** schematically shows two embodiments of a medical apparatus comprising a hadron therapy device coupled to a MRI;

**Figure 4** schematically illustrates the hadron pencil beam treatment of a target tissue;

**Figure 5** schematically shows a selection of an imaging slice, Vpi, in a MRI and creation of phase gradients and frequency gradients;

**Figure 6** shows two examples of an apparatus according to the present invention, showing access of a hadron beam to a target tissue;

**Figure 7** shows an example of a medical apparatus comprising a hadron therapy device, a MRI device, and a hadron radiography system according to the present invention;

**Figure 8** schematically illustrates examples of detectors for a hadron radiography system;

**Figure 9** schematically illustrates the computation of the energy of a hadron beam according to the present invention;

**Figure 10&11** show flowcharts of an hadron therapy methods using a medical apparatus according to the present invention;

**Figure 12** shows an embodiment of a medical apparatus according to the present invention and comprising a hadron therapy device, a MRI device, a hadron radiography system, and a prompt-gamma detector or a PET scan;

**Figure 13** shows an embodiment of a medical apparatus according to the present invention and comprising a hadron therapy device, a MRI device, a hadron radiography system, and a support for supporting a patient in a non supine position.

The figures are not drawn to scale. Generally, identical components are denoted by the same reference numerals in the figures.

**Detailed description of preferred embodiments**

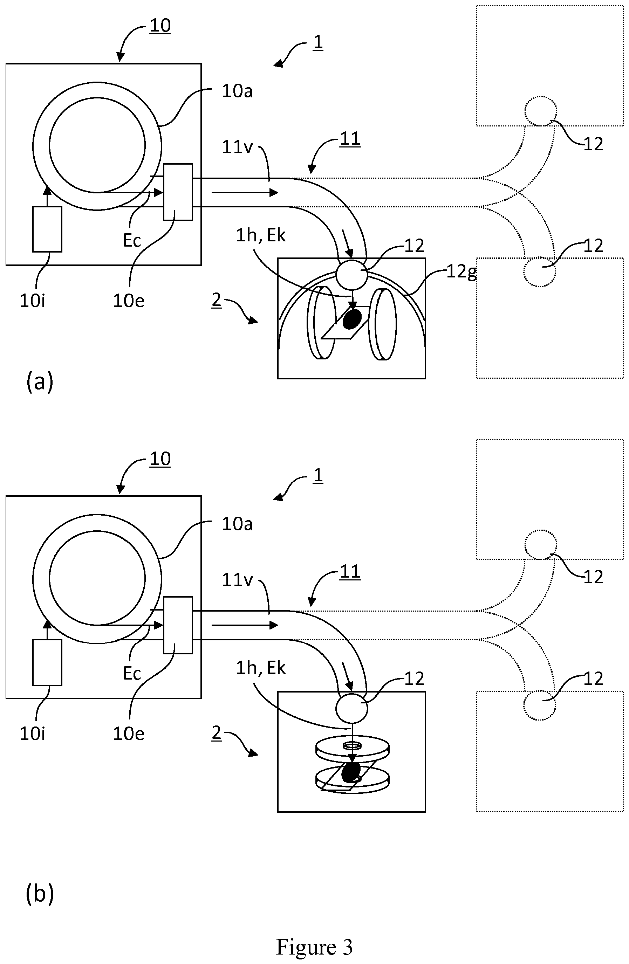

Hadron therapy is a form of external beam radiotherapy using beams **1h** of energetic hadrons. Figures 3, 4&6 show a hadron beam **1h** directed towards a target spot **40s** in a target tissue **40** of a subject of interest. Target tissues **40** of a subject of interest typically include cancerous cells forming a tumour. During a hadron therapy session, a hadron beam of initial energy, **Ek,** with k = 0 or 1, irradiates one or more target spots within the target tissue, such as a tumour, and destroys the cancerous cells included in the irradiated target spots, thus reducing the size of the treated tumour by necrosis of the irradiated tissues.

The subject of interest may comprise a plurality of materials including organic materials. Preferably, the subject of interest comprises a plurality of tissues **m,** with m = 40-44 as shown in Figure 2, that can be, for example: skin, fat, muscle, bone, air, water (blood), organ, and tumour. The target tissue **40** is preferably a tumour.

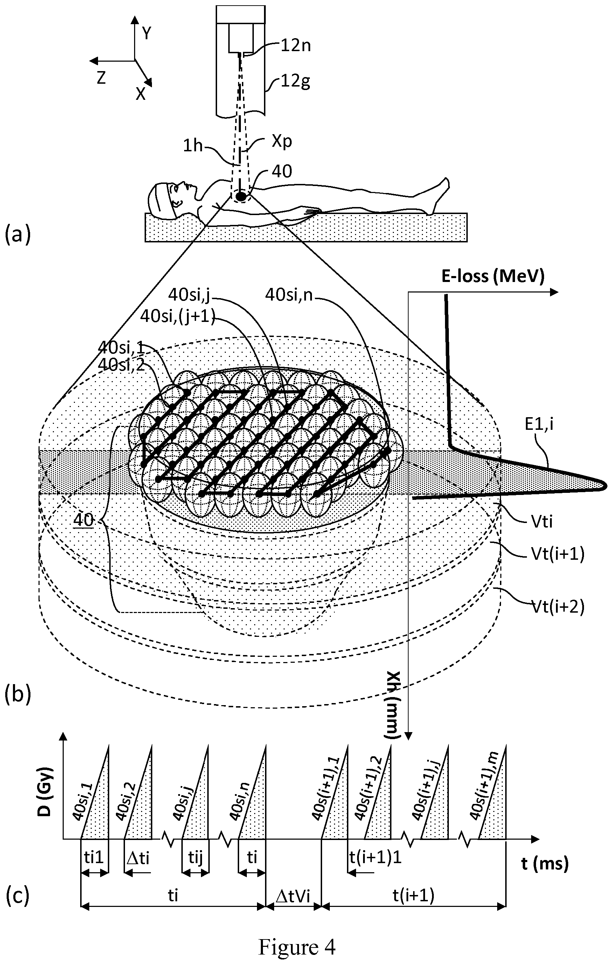

As described in the prior art literature, a hadron beam **1h** traversing an organic body along a beam path, **Xp,** loses most of its energy at a specific distance of penetration along the beam path, **Xp.** As illustrated in Figures 2&4, said specific distance of penetration corresponds to the position of the so called Bragg peak, observed when plotting the energy loss per unit distance [MeVg$^{-1}$cm$^{-2}$], **E$_{loss}$,** of a hadron beam as a function of the distance, **xh,** measured along the beam path, **Xp.** Unlike other forms of radiation therapies, a hadron beam can thus deliver a high dose of energy at a very specific location within a target tissue corresponding to the position of the Bragg peak. The position of the Bragg peak depends mainly on the initial energy, **Ek,** of the hadron beam (i.e., before traversing any tissue) and on the nature and thicknesses of the traversed tissues. The hadron dose delivered to a target spot depends on the intensity of the hadron beam and on the time of exposure. The hadron dose is measured in Grays (Gy), and the dose delivered during a treatment session is usually of the order of one to several Grays (Gy).

A hadron is a composite particle made of quarks held together by strong nuclear forces. Typical examples of hadrons include protons, neutrons, pions, and heavy ions, such as carbon ions. In hadron therapy, electrically charged hadrons are generally used. Preferably, the hadron is a proton and the corresponding hadron therapy is referred to as proton therapy. In the following, unless otherwise indicated, any reference to a proton beam or proton therapy applies to a hadron beam or hadron therapy in general.

A hadron therapy device **1** generally comprises a hadron source **10,** a beam transport line **11,** and a beam delivery system **12.** Charged hadrons may be generated from an injection system **10i,** and may be accelerated in a particle accelerator **10a** to build up energy. Suitable accelerators include for example, a cyclotron, a synchro-cyclotron, a synchrotron, or a laser accelerator. For example, a (synchro-)cyclotron can accelerate charged hadron particles from a central area of the (synchro-)cyclotron along an outward spiral path until they reach the desired output energy, **Ec,** whence they are extracted from the (synchro-)cyclotron. Said output energy, **Ec,** reached by a hadron beam when extracted from the (synchro-)cyclotron is typically comprised between 60 and 400 MeV, preferably between 210 and 250 MeV. The output energy, **Ec,** is not necessarily the initial energy, **Ek,** of the hadron beam used during a therapy session; **Ek** is equal to or lower than **Ec, Ek $\leq$ Ec.** An example of a suitable hadron therapy device includes, but is not limited to, a device described in US Pat. No. 4870287, the entire disclosure of which is incorporated herein by reference as representative of a hadron beam therapy device as used in the present invention.

The energy of a hadron beam extracted from a (synchro-)cyclotron can be decreased by energy selection means **10e** such as energy degraders, positioned along the beam path, **Xp,** downstream of the (synchro-)cyclotron, which can decrease the output energy, **Ec,** down to any value of **Ek** including down to nearly 0 MeV. As discussed supra, the position of the Bragg peak along a hadron beam path, **Xp,** traversing specific tissues depends on the initial energy, **Ek,** of the hadron beam. By selecting the initial energy, **Ek,** of a hadron beam intersecting a target spot **40s** located within a target tissue, the position of the Bragg peak can be controlled to correspond to the position of the target spot.

A hadron beam can also be used for characterizing properties of tissues. For example, images can be obtained with a hadron radiography system (HRS or, in particular a proton radiography system, PRS). The doses of hadrons delivered to a target spot for characterization purposes, however, may be considerably lower than the doses delivered during a hadron therapy session which, as discussed supra, are of the order of 1 to 10 Gy. The doses of delivered hadrons of HRS for characterization purposes are typically of the order of $10^{-3}$ to $10^{-1}$ Gy (i.e. one to four orders of magnitude lower than doses typically delivered for therapeutic treatments). These doses have no significant therapeutic effects on a target spot. Alternatively, a treatment hadron beam delivered to a small set of target spots in a target tissue may be used for characterization purposes. The total dose delivered for characterization purposes is not sufficient to treat a target tissue.

As illustrated in Figure 3, downstream of the hadron source, a hadron beam of initial energy, **Ek,** is directed to the beam delivery system **12** through a beam transport line **11.** The beam transport line may comprise one or more vacuum ducts, **11v,** a plurality of magnets for controlling the direction of the hadron beam and/or for focusing the hadron beam. The beam transport line may also be adapted for distributing and/or selectively directing the hadron beam from a single hadron source **10** to a plurality of beam delivery systems for treating several patients in parallel.

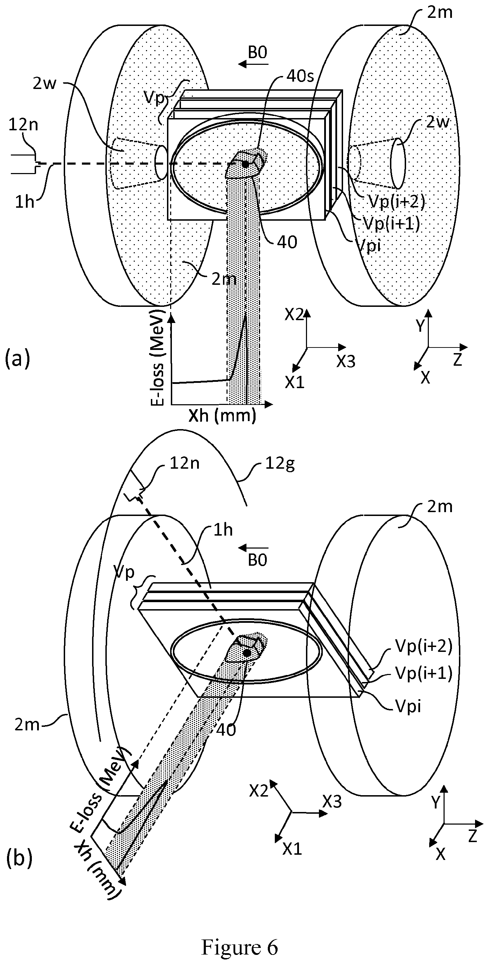

The beam delivery system **12** comprises a nozzle **12n** for orienting a hadron beam **1h** along a beam path, **Xp.** The nozzle can either be fixed or mobile. Mobile nozzles are generally mounted on a gantry **12g** as illustrated schematically in Figures 4&6. A gantry is used for varying the orientation of the hadron outlet about a circle centred on an isocentre and normal to an axis, **Z,** which is generally horizontal. In supine hadron treatment devices, the horizontal axis, **Z,** may be selected parallel to a patient lying on a couch (i.e. the head and feet of the patient are aligned along the horizontal axis, **Z).** The nozzle **12n** and the isocentre define a path axis, **Xn,** which angular orientation depends on the angular position of the nozzle in the gantry. By means of magnets positioned adjacent to the nozzle, the beam path, **Xp,** of a hadron beam **1h** can be deviated with respect to the path axis, **Xn,** within a cone centred on the path axis and having the nozzle as apex (cf. Figure 4(a)). This is advantageous in that a volume of target tissue centred on the isocentre can be treated by hadron beam without changing the position of the nozzle within the gantry. The same applies to fixed nozzles with the difference that the angular position of the path axis is fixed.

A target tissue to be treated by a hadron beam in a device provided with a gantry must be positioned near the isocentre. To this purpose, the couch or any other support for the patient can be moved; it can typically be translated over a horizontal plane **(X, Z)** wherein **X** is a horizontal axis normal to the horizontal axis, **Z,** and translated over a vertical axis, **Y,** normal to **X** and **Z,** and can also be rotated about any of the axes **X, Y, Z,** so that a central area of the target tissue can be positioned at the isocentre.

To assist in the correct positioning of a patient with respect to the nozzle **12n** according to a treatment plan previously established, the beam delivery system may comprise imaging means. For example, a conventional X-ray radiography system can be used to image an imaging volume, **Vp,** comprising the target tissue **40.** The thus obtained images can be compared with corresponding images collected previously during the establishment of the treatment plan.

Depending on the pre-established treatment plan, a hadron treatment may comprise delivery of a hadron beam to a target tissue in various forms, including the following techniques well known in the art: pencil beam, single scattering, double scattering, and uniform scattering. The present invention may apply to all hadron therapy technique. The hadron treatment, however, is preferably applied by a pencil beam technique. Figure 4 illustrates schematically this technique of delivery. A hadron beam of initial energy, **Ek,1,** is directed to a first target spot **40s1,1,** during a pre-established delivery time. The hadron beam is then moved to a second target spot **40s1,2,** during a pre-established delivery time. The process is repeated on a sequence of target spots **40s1,j** to scan a first iso-energy treatment volume, **Vt1,** following a pre-established scanning path. A second iso-energy treatment volume, **Vt2,** is scanned spot by spot following a similar scanning path with a hadron beam of initial energy, **Ek,2.** As many iso-energy treatment volumes, **Vti,** as necessary to treat a given target tissue **40** are thus irradiated following a similar scanning path. A scanning path can include several passages over a same scanning spot **40si,j.** The iso-energy treatment volumes, **Vti,** are volumes of target tissues which can be treated with a hadron beam of initial energy, **Ek,i.** The iso-energy treatment volumes, **Vti,** are slice shaped, with a thickness corresponding approximately to the breadths of the Bragg peaks at the values of the initial energy, **Ek,i,** of the corresponding hadron beams, and with main surfaces of area only limited by the opening angle of the cone centred on the path axis, **Xn,** enclosing the beam paths, **Xp,** available for a given position of the nozzle in the gantry or in a fixed nozzle device. In case of a homogeneous target tissue, the main surfaces are substantially planar as illustrated in Figure 4(b). In reality, however, since both target tissue **40** and upstream tissues **41-43** are not homogeneous in nature and thickness, the main surfaces of an iso-energy volume, **Vti,** are bumpy. The egg-shaped volumes in Figure 4(b) schematically illustrate the volumes of target tissue receiving a therapeutic dose of hadron by exposure of one target spot **40si,j** to a beam of initial energy **Ek,i.**

The dose, **D,** delivered to a target tissue **40** is illustrated in Figure 4(c). As discussed supra, the dose delivered during a treatment session is usually of the order of one to several Grays (Gy). It depends on the doses delivered to each target spot **40si,j,** of each iso-energy treatment volume, **Vti.** The dose delivered to each target spot **40si,j** depends on the intensity, **I,** of the hadron beam and on the irradiation time **tij** on said target spot. The dose, **Dij,** delivered to a target spot **40si,j** is therefore the integral, $\mathbf{Dij} = \int I\,dt$, over the irradiation time **tij.** A typical dose, **Dij,** delivered to a target spot **40si,j** is of the order of 0.1-20 cGy. The dose, **Di,** delivered to an iso-energy treatment volume, **Vti,** is the sum over the n target spots scanned in said iso-energy treatment volume of the doses, **Dij,** delivered to each target spot, $\mathbf{Di} = \sum \mathbf{Dij}$, for **j** = 1 to n. The total dose, **D,** delivered to a target tissue **40** is thus the sum over the **p** irradiated iso-energy treatment volumes, **Vti,** of the doses, **Di,** delivered to each energy treatment volume, $\mathbf{D} = \sum Di$, for i = 1 to p. The dose, **D,** of hadrons delivered to a target tissue can therefore be controlled over a broad range of values by controlling one or more of the intensity, **I,** of the hadron beam, the total irradiation time **tij** of each target spot **40si,j,** and the number of irradiated target spots **40si,j.** Once a patient is positioned such that the target tissue **40** to be treated is located at the approximate position of the isocentre the duration of a hadron treatment session therefore depends mainly on the values of:

- the irradiation time, **tij,** of each target spot **40si,j,**

- the scanning time, Δ**ti,** for directing the hadron beam from a target spot **40si,j** to an adjacent target spot **40si(j+1)** of a same iso-energy treatment volume, **Vti,**

- the number n of target spots **40si,j** scanned in each iso-energy treatment volume, **Vti,**

- the time, Δ**tVi,** required for passing from a last target spot **40si,n** scanned in an iso-energy treatment volume, **Vti,** to a first target spot **40s(i+1),1** of the next iso-energy treatment volume, **Vt(i+1),** and

- the number of iso-energy treatment volumes, **Vti,** in which a target tissue **40** is enclosed.

The irradiation time, **tij,** of a target spot **40si,j** is of the order of 1-20 ms. The scanning time, Δ**ti,** between successive target spots in a same iso-energy treatment volume is generally very short, of the order of 1 ms. The time, Δ**tVi,** required for passing from one iso-energy treatment volume, **Vti,** to a subsequent iso-energy treatment volume, **Vt(i+1),** is slightly longer because it requires changing the initial energy, **Ek,** of the hadron beam and is of the order of 1-2 s.

As illustrated in Figure 2(a)&(b), an accurate determination of the initial energy, **Ek,** of a hadron beam is clearly critical, because if the position of the Bragg peak thus determined does not correspond to the actual position of the target tissue **40,** substantial doses of hadrons could be delivered to healthy, sometimes vital organs and could possibly endanger the health of a patient. The position of the Bragg peak mainly depends on the initial energy, **Ek,** of the hadron beam and on the nature and thicknesses of the traversed tissues. Besides determining the exact position of the target tissue within a patient, the computation of the initial energy, **Ek,** of a hadron beam yielding a position of the Bragg peak corresponding to the precise position of the target tissue therefore also requires the preliminary characterization of the tissues traversed until reaching the target tissue **40.** This characterization is performed during a treatment plan established before (generally several days before) the actual hadron treatment. The actual hadron treatment can be divided in several sessions distributed over several weeks. A typical treatment plan may start by the acquisition of data, generally in the form of images of the subject of interest with a CT scan. The images thus acquired by a CT scan may be characterized, by performing one or more of the steps:

- identifying the nature of the tissues represented on the images as a function of the X-rays absorption power of the tissues, based on the comparison of shades of grey of each tissue with a known grey scale; for example, a tissue can be one of fat, bone, muscle, water, air;

- measuring the positions and thicknesses of each tissue along one or more hadron beam paths, **Xp,** from the skin to the target tissue;

- based on their respective nature, attributing to each identified tissue a corresponding hadron stopping power ratio (HSPR);

- calculating a tissue water equivalent path length, **WEPLm,** of each tissue **m,** with m = 40 to 44, upstream of and including the target tissue, based on their respective HSPR and thicknesses;

- adding the thus determined **WEPLm** of all tissues **m** to yield a **WEPL40s,** of a target spot **40s** located in the target tissue **40,** said **WEPL40s** corresponding to the distance travelled by hadron beam from the skin to the target spot **40s;**

- from the **WEPL40s,** calculating the initial energy **Ek** of a hadron beam required for positioning the Bragg peak of the hadron beam at the target spot **40s.**

Said process steps can be repeated for several target spots defining the target tissue.

**Magnetic resonance imaging device**

A magnetic resonance imaging device **2** (MRI) implements a medical imaging technique based on the interactions of excitable atoms present in an organic tissue of a subject of interest with electromagnetic fields. When placed in a strong main magnetic field, **B0,** the spins of the nuclei of said excitable atoms precess around an axis aligned with the main magnetic field, **B0,** resulting in a net polarization at rest that is parallel to the main magnetic field, **B0.** The application of a pulse of radio frequency (RF) exciting magnetic field, **B1,** at the frequency of resonance, **fL,** called the Larmor frequency, of the excitable atoms in said main magnetic field, **B0,** excites said atoms by tipping the net polarization vector sideways (e.g., with a so-called 90° pulse, **B1-90**) or to angles greater than 90° and even reverse it at 180° (with a so-called 180° pulse, **B1-180**). When the RF electromagnetic pulse is turned off, the spins of the nuclei of the excitable atoms return progressively to an equilibrium state yielding the net polarization at rest. During relaxation, the transverse vector component of the spins produces an oscillating magnetic field inducing a signal which can be collected by antennas **2a** located in close proximity to the anatomy under examination.

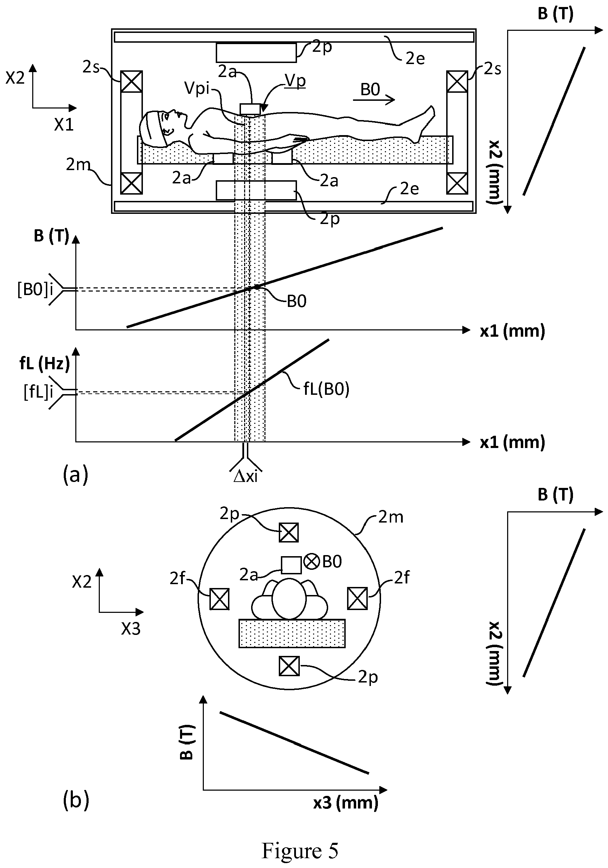

As shown in Figures 5 and 6, a MRI **2** usually comprises a main magnet unit **2m** for creating a uniform main magnetic field, **B0;** radio frequency (RF) excitation coils **2e** for creating the RF-exciting magnetic field, **B1;** X1-, X2-, and X3-gradient coils, **2s, 2p, 2f,** for creating magnetic gradients along the first, second, and third directions **X1, X2,** and **X3,** respectively; and antennas **2a,** for receiving RF-signals emitted by excited atoms as they relax from their excited state back to their rest state. The main magnet produces the main magnetic field, **B0,** and can be a permanent magnet or an electromagnet (a supra-conductive magnet or not). An example of a suitable MRI includes, but is not limited to, a device described inEP Pat. No. 0186238, the entire disclosure ofwhich is incorporated herein by reference.

As illustrated in Figure 5, an imaging slice or layer, **Vpi,** of thickness, **Δxi,** normal to the first direction, **X1,** can be selected by creating a magnetic field gradient along the first direction, **X1.** In Figure 5, the first direction, **X1,** is parallel to the axis Z defined by the lying position of the patient, yielding slices normal to said axis Z. In practice, this is not necessarily the case, and the first direction, **X1,** can be any direction, e.g. transverse to the axis Z, with slices extending at an angle with respect to the patient. As shown in Figure 5(a), because the Larmor frequency, **fL,** of an excitable atom depends on the magnitude of the magnetic field it is exposed to, sending pulses of RF exciting magnetic field, **B1,** at a frequency range, **[fL]i,** excites exclusively the excitable atoms which are exposed to a magnetic field range, **[B0]i,** which are located in a slice or layer, **Vpi,** of thickness, **Δxi.** By varying the frequency bandwidth, **[fL]i,** of the pulses of RF exciting magnetic field, **B1,** the width, **Δxi,** and position of an imaging layer, **Vpi,** can be controlled. By repeating this operation on successive imaging layers, **Vpi,** an imaging volume, **Vp,** can be characterized and imaged.

To localize the spatial origin of the signals received by the antennas on a plane normal to the first direction, **X1,** magnetic gradients are created successively along second and third directions, **X2, X3,** wherein X1 ⊥ X2 ⊥ X3, by activating the X2-, and X3-gradient coils **2p, 2f,** as illustrated in Figure 5(b). Said gradients provoke a phase gradient, Δφ, and a frequency gradient, Δ**f,** in the spins of the excited nuclei as they relax, which allows spatial encoding of the received signals in the second and third directions, **X2, X3.** A two-dimensional matrix is thus acquired, producing *k*-space data, and an MR image is created by performing a two-dimensional inverse Fourier transform. Other modes of acquiring and creating an MR image are known in the art and the present invention is not restricted to the selection of any particular mode.

The main magnetic field, **B0,** is generally comprised between 0.2 and 7 T, preferably between 1 and 4 T. The radiofrequency (RF) excitation coils **2e** generate a magnetic field at a frequency range, **[fL]i,** around the Larmor frequencies, **fL,** of the atoms comprised within a slice of thickness, $\Delta$**xi,** and exposed to a main magnetic field range **[B0i].** For atoms of hydrogen, the Larmor frequency per magnetic strength unit, **fL** / **B** = 42.6 MHz T$^{-1}$. For example, for hydrogen atoms exposed to a main magnetic field, **B0** = 2 T, the Larmor frequency, **fL** = 85.2 MHz.

The MRI can be any of a closed-bore, open-bore, or wide-bore MRI type. A typical closed-bore MRI has a magnetic strength of 1.0 T through 3.0 T with a bore diameter of the order of 60 cm. An open-bore MRI, as illustrated in Figure 6, has typically two main magnet poles **2m** separated by a gap between them for accommodating a patient in a lying position, sitting position, or any other position suitable for imaging an imaging volume, **Vp.** The magnetic field of an open-bore MRI is usually comprised between 0.2 and 1.0 T. A wide-bore MRI is a kind of closed-bore MRI having a larger diameter.

## Hadron therapy device + MRI

As discussed in the introduction with reference to Figure 2(b), the position and morphology of a target tissue **40** can evolve between a time, **t0,** of establishment of a treatment plan and a time, **t1** = **t0** + $\Delta$**t3,** of a treatment session, which can be separated by several days or weeks. A target spot **40si,j** identified in the treatment plan as belonging to the target tissue **40p** may not belong to the target tissue **40** anymore at the time, **t0** + $\Delta$**t3,** of the treatment session. The irradiation of said target spot may harm healthy tissues **43** instead of target tissues **40.**

To avoid such incidents, the prior art proposes coupling a hadron therapy device (PT) 1 to an imaging device, such as a magnetic resonance imaging device (MRI) **2.** Such coupling may not be trivial with a number of challenges to overcome but PT-MRI apparatuses have been described in the recent art and are generally known by the persons of ordinary skill in the art. For example, solutions to problems such as the correction of a hadron beam path, **Xp,** within a strong magnetic field, **B0,** of the MRI are available.

A PT-MRI apparatus allows the morphologies and positions of the target tissue and surrounding tissues to be visualized the day, **t0** + $\Delta$**t3,** of the treatment session for comparison with the corresponding morphologies and positions acquired during the establishment of a treatment plan at time, **t0.** As illustrated in the flowchart of Figure 1, in case a discrepancy of the tissues morphologies and positions between the establishment of the treatment plan at time, **t0,** and the treatment session at time, **t0** + $\Delta$**t3,** was observed, the treatment session would be interrupted and a new treatment plan would probably be established with the definition of new target spots corresponding to the actual target tissue **40** to be irradiated by hadron beams of corrected energies and directions (cf. Figure 1, diamond box "$\exists \Delta$?" $\rightarrow$ Y $\rightarrow$ "STOP"). This development of the prior art represents already a major improvement over carrying out a hadron therapy session based solely on information collected during the establishment of the treatment plan at time, **t0,** which may be obsolete at the time, **t0** + $\Delta$**t3,** of the treatment session.

The present invention aims at further improving the efficacy of a PT-MRI apparatus by providing the information required for *correcting in situ* the initial energies, **Ek,** and beam path, **Xp,** directions of the hadron beams, in case a change of morphology or position of the target tissue were detected. This would allow the treatment session to take place in spite of any changes detected in the target tissue **40.**

The MRI used can be any of a closed-bore, open-bore, or wide-bore MRI type described above. An open MRI affords much open space in the gap separating the two main magnet poles **2m** for orienting a hadron beam in almost any direction. Alternatively, openings or windows **2w** transparent to hadrons can be provided on the main magnet units as illustrated in Figure 6(a). This configuration has the particularity that the hadron beam can be parallel to B0. In another embodiment, a hadron beam can be oriented through the cavity of the tunnel formed by a closed bore MRI, or an annular window transparent to hadrons may extend parallel to a gantry substantially normal to the axis Z, over a wall of said tunnel, such that hadron beams may reach a target tissue with different angles. In case a fixed nozzle is used, the size of such opening or window can be reduced accordingly.

## Hadron radiography system

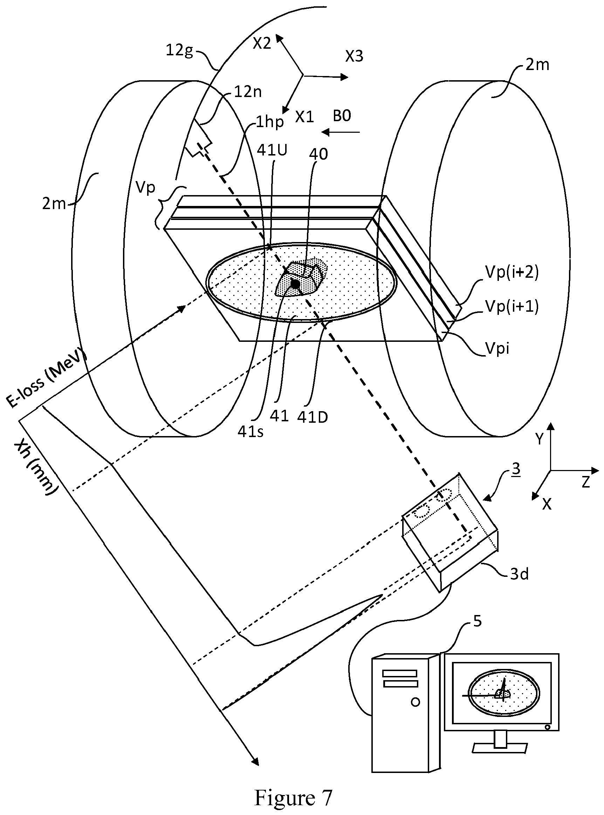

Figure 7 illustrates an example of a medical apparatus comprising a hadron therapy device **1,** a magnetic resonance imaging device (MRI) **2,** a hadron radiography system (HRS), **3,** and a controller **5.**

HRS comprises a detector **3d** configured for detecting a signal generated by an imaging hadron beam **1hp.** The imaging hadron beam has an initial imaging beam energy, **E0p,** usually higher than the energy of a treatment hadron beam used to treat a target tissue **40.** The initial imaging beam energy, **E0p,** has to be high enough to go through and beyond a subject of interest. For example, the initial imaging beam energy, **E0p,** may be higher than 100 MeV per nucleon, preferably higher than 120 MeV per nucleon, more preferably higher than 150 MeV per nucleon. The intensity of the imaging hadron beam is lower than the intensity of a treatment hadron beam. The imaging hadron beam can be directed towards one or more target spots **40si,j** located inside the target tissue **40.** The total dose delivered to the target spot(s) by the imaging hadron beam may be lower than $10^{-3}$ to $10^{-1}$ Gy, preferably, the total dose is lower than $10^{-2}$ Gy. The imaging hadron beam **1hp** follows a beam path coming from the nozzle **12n** of the beam delivery system. The beam path of the imaging hadron beam at least crosses:

(A) an upstream boundary **41U** of the subject of interest, for example the skin of a patient;

(B) the target spot **40s** within the target tissues **40** inside of the subject of interest; and

(C) a downstream boundary **41D** of the subject of interest, for example the skin of a patient.

The terms "upstream" and "downstream" are defined with respect to the direction of the hadron beam.

A hadron beam that crosses material loses a part of its energy all along its beam path. The loss is due to the interactions of the hadrons with the electrons of the tissues **m,** with m= 41-43, traversed (Coulomb interaction) and to the interactions with the atomic nuclei of the tissues traversed (Coulomb interaction or elastic collision). The loss is proportional to the thickness **Lm** of the tissue **m** traversed and depends on the nature of the tissue m. In particular, the loss depends on the density of the tissue m traversed by the hadron beam. In hadron therapy, the tissue traversed by a hadron beam are, for example, skin, fat, muscle, bone, air, water (blood), organ, and tumour. The total attenuation of the (imaging) hadron beam is equal to the sum of the attenuation in each tissue. The attenuation in the air before the upstream boundary and after the downstream boundary is usually negligible.

The detector **3d** of the HRS measures the residual energy or, equivalently, the residual range of the imaging hadron beam. The residual energy is the remaining energy of the hadron beam after crossing the subject of interest. The residual range is the distance in water that a hadron beam can cross before the Bragg peak.

Knowing the initial beam energy, **E0p,** and the residual energy in the detector, the controller 5 can compute the energy lost by the hadron beam within the portion of the subject of interest comprised between the upstream and downstream boundaries. From the energy lost, the controller can compute a Bragg curve such as illustrated in Figure 2(c). The loss of energy corresponds to a water equivalent path length, **WEPL,HRS,** of a portion of the subject of interest comprised between the upstream and downstream boundaries.

The detector **3d** of the HRS can be one of the following detector: a range telescope **3t,** a calorimeter **3c** or a spectrometer **3s.** The detector is located opposite to the nozzle with respect to the isocenter, and is crossed by the beam path of the imaging hadron beam. The detector can be located inside or outside of the MRI. The detector can be mounted on the gantry of the hadron delivery system, or on a dedicated gantry. Alternatively, the position of the detector may be fixed.

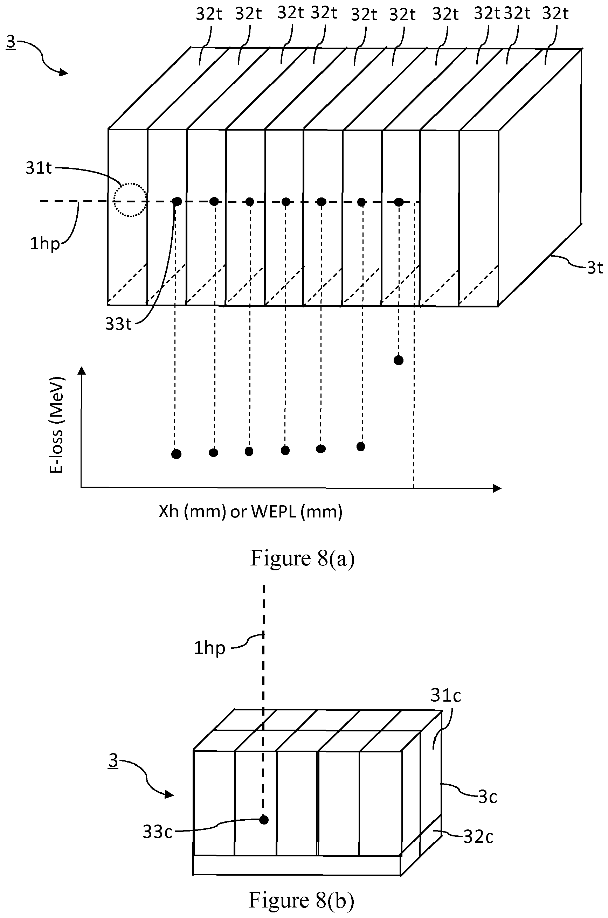

A range telescope is a detector that allows measuring the range of the particles following a defined trajectory. Range telescope designs include stacks of scintillators and multi-layer ionisation chambers. Figure 8(a) illustrates a range telescope comprising multi-layer ionisation chambers **32t.** The hadron beam **1h** goes into the detector through an aperture **31t,** the energy of the imaging hadron beam is then measures in each ionisation chambers thus giving measurement points **33t.** These measurement points are used to compute a curve, **34t,** of the energy lost in function of the distance in the detector (which can be water equivalent or not).

A calorimeter is a detector that measures the energy of a particle by letting the particle totally release its energy inside the device and measuring this amount of energy. Figure 8(b) shows an example of calorimeter **3c** comprising an absorbing material **31c** that absorbs the residual energy of the imaging hadron beam and a measurement means **32c** that can measure the energy absorbed by the absorbing material (for example by measuring an increase of temperature of the absorbing material). The detector can be segmented to obtain information on the orientation.

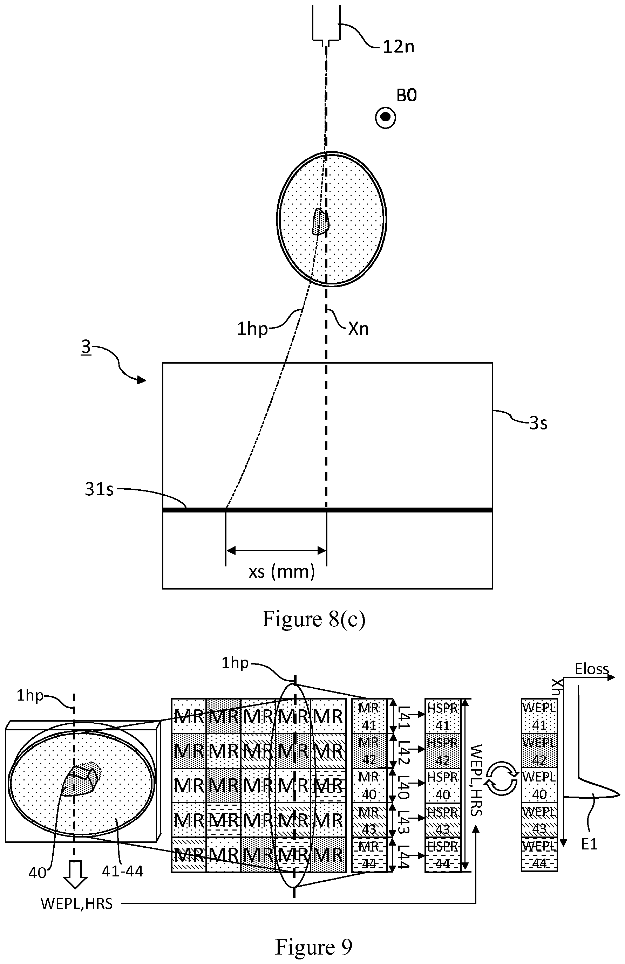

A spectrometer is a detector that measures the energy distribution of an imaging hadron. The detector measures the number of particles within each energy range. Figure 8(c) illustrates an example of spectrometer **3s.** A spectrometer has to comprise a dispersive element. For example, the dispersive element of an optical spectrometer can be a prism. The dispersive element of a charged hadron spectrometer can be the main magnetic field **B0** of the MRI. In the case where the charged hadron beam is normal (or at least not parallel) to **B0,** it is deviated according to the Lorentz law. The deviation is proportional to the velocity (or, equivalently, the energy) of the hadron beam. For example, the deviation is larger for lower energy. The imaging hadron beam loses energy as it crosses tissues inside the subject of interest, thus leading to a larger deviation. By measuring the distance, **xs,** from a reference point, for example, the intersection of the path axis, **Xn,** with a detecting plate **31s** of the spectrometer, to the point of the detecting plate **31s** intersected by the imaging hadron beam, a controller can compute the (residual) energy of the imaging hadron beam. The distance, **xs,** depends on the (residual) energy of the hadron beam and allows the computation of the WEPL of the tissue traversed by the hadron beam. Practically, the residual energy is computed following an iterative process. The water equivalent path length **WEPL,HRS** is estimated from the MR data and the deviation of the hadron beam is simulated. Then, the position of the simulated hadron beam on the detector of the spectrometer is compared to the actual position measured. The **WEPL,HRS** is then corrected and a hadron beam is again simulated until the simulated position is equal to the measured position thus leading to the correct **WEPL,HRS.**

The medical apparatus according to the present invention comprises:

(A) a hadron therapy device comprising a hadron source **10** adapted for directing an imaging hadron beam **1hp** having an initial imaging beam energy, **E0p,** along a beam path, said beam path going through and beyond:

- a subject of interest crossed by the beam path between upstream and downstream boundaries, and

- a target spot **40s** located inside a target tissue of the subject of interest between the upstream and downstream boundaries;

(B) a MRI for acquiring magnetic resonance (MR) data within an imaging volume, **Vp,** comprising a portion of the subject of interest including the target spot and at least a portion of the beam path comprised between the upstream boundary and the target spot;

(C) a HRS adapted for acquiring a signal generated by the imaging hadron beam; and

(D) a controller **5** configured for determining a water equivalent path length **WEPL,HRS** of the beam path between upstream and downstream boundaries, said determination being based, at least, on the signal acquired with the HRS.

The position and morphology of a target tissue **40** can evolve between a time, **t0,** of establishment of a treatment plan and a time, **t0 + $\Delta$t3,** of a treatment session. A PT-MRI apparatus allows the morphologies and positions of the target tissue and surrounding tissues to be visualized the day, **t0 + $\Delta$t3,** of the treatment session for comparison with the corresponding morphologies and positions acquired during the establishment of a treatment plan at time, **t0.** Images from MRI do not allow to accurately determine the WEPL and/or the HSPR and in consequence, the treatment plan cannot be adapted during a treatment session. HRS allows to measure the WEPL of an imaging hadron beam and, from that, to correct and adapt the treatment plan during a treatment session. The hadron therapy device according to the present invention thus allows a correction of the energies and directions of the hadron beams in case a change of morphologies or positions of the target tissues is detected during the treatment session. It thus reduces the risk of irradiating healthy tissue or missing target tissue that leads to the formation of new cancer. It also improves the efficiency of the treatment in allowing the adaptation of the dose delivered at a target spot.

Preferably, the MR data provided by the MRI and the signal generated by the hadron beam provided by the HRS are acquired simultaneously or with a short delay. The two measures are thus representative of the same configuration of the tissues.

Advantageously, the plan of the MR image comprises the beam path of the (imaging) hadron beam. The MR image can be used to (help to) determine the nature of the tissues **m,** with m = 40 to 44, traversed by the hadron beam and to determine the thicknesses **Lm** of the tissues **m** traversed by the hadron beam. It is therefore useful to image the plan in which the imaging hadron beam passes.

The controller **5** can be configured for computing the water equivalent path length, **WEPLm,** of tissues **m,** of thickness **Lm,** with m = 40 to 44, crossed by the beam path and comprised between the upstream and downstream boundaries. As illustrated on Figure 9, MR data are acquired by the MRI. The MR data correspond to the density of hydrogen atoms within the tissues imaged. The tissue traversed by the imaging hadron beam can be selected on the MR data. The controller can determine or estimate:

- the nature of the tissues **m** traversed by the imaging hadron beam;

- an **HSPR,m** of each tissue **m;**

- the thickness, **Lm** of the tissues **m.**

By equating the thickness **Lm,** the **HSPR,m** and the **WEPL,HRS** measured with the HRS, the controller can compute the water equivalent path lengths, **WEPLm's,** of each tissues **m.** This computation can be, for example, an iterative process leading to an optimisation of the value of the **Lm** and of the **HSPR,m** with respect to the measured **WEPL,HRS.**

The controller may then calculate a value of the water equivalent path length **WEPL40s,k** with k = 0 or 1, of a beam path comprised between the upstream boundary and the target spot, said calculation being based on the **WEPLm's** of the tissues **m** positioned upstream of and including the target tissue **40.** The **WEPL40s,k** is used to compute an initial energy **Ek** of a treatment hadron beam such that the position of the Bragg peak of the treatment hadron beam, measured along the beam path, corresponds to the position, **Pk,** of the target spot **40s.**

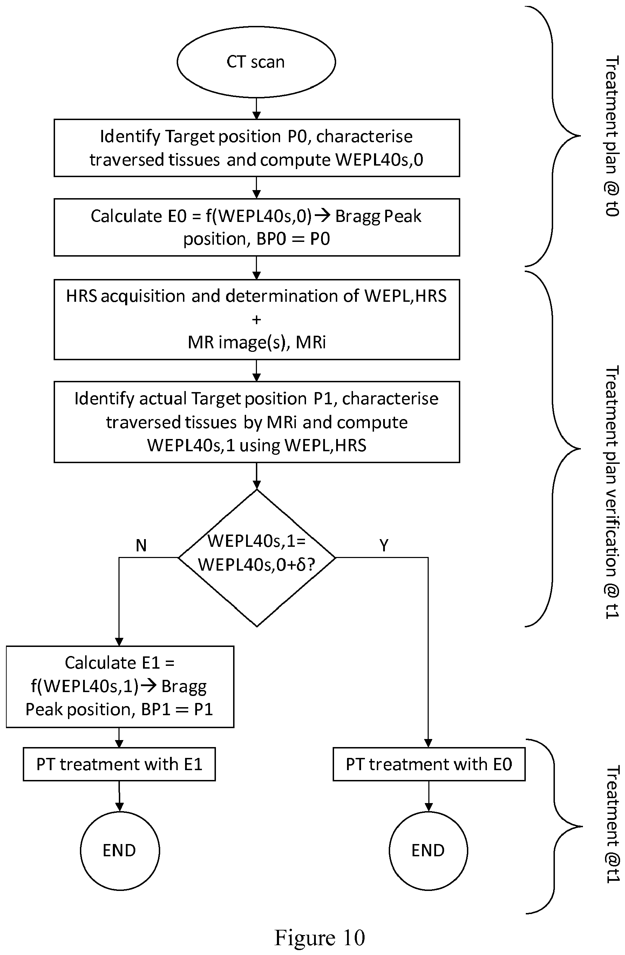

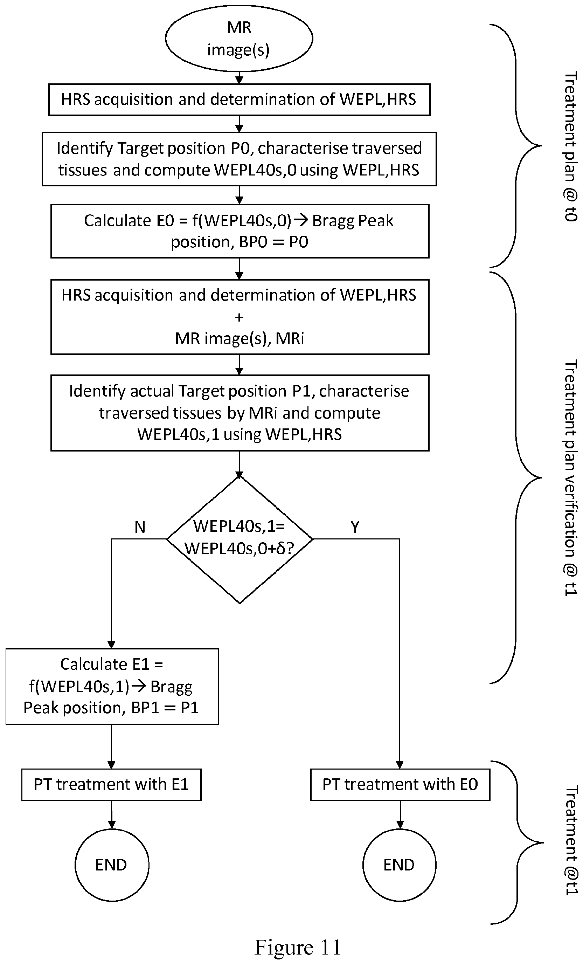

Figure 10 and 11 illustrate examples of work flows of a hadron therapy using the apparatus according to the invention. First, a treatment plan can be established either classically as described above with a CT scan (Figure 10) or with the medical apparatus according to the present invention (Figure 11). Second, during a treatment session, a treatment plan verification can be performed with the apparatus according to the present invention, and third the treatment can be applied. Several treatment sessions are usually delivered.

The treatment plan provides a planned initial energy, **E0,** of a treatment hadron beam based on the computation of a water equivalent path length, **WEPL40s,0,** of a beam path comprised between the upstream boundary and the target spot **40s.** The treatment plan can comprise the computation of several planned initial energies, **E0i,j** based on the computation of water equivalent path lengths, **WEPL40si,j,0** corresponding to several target spots **40si,j** belonging to the target tissue **40.** The treatment plan is usually done at a time t0, several days or weeks before the treatment sessions.

As illustrated in Figure 11, the **WEPL40s,0** can be computed with the controller **5** of the medical apparatus according to the invention. The controller 5 acquires MR images and **WEPL,HRS.** MR images are used to estimate the nature (including the density), the thickness **Lm** and the **HSPR,m** of the tissues traversed by the imaging hadron beam. These data are equating with the **WEPL,HRS** measured with the HRS to compute water equivalent path lengths **WEPLm's** of tissues **m** that are then used to compute the **WEPL40s,0.** The value of the **WEPL40s,0** allows the controller to compute a planned initial energy, **E0,** of a treatment hadron beam. The computation yields the Bragg peak at a planned position, **BP0,** corresponding to the position of the target spot, **P0,** during the establishment of the treatment plan. Alternatively, as illustrated on Figure 10, the **WEPL40s,0** can be computed from a CT scan image.

For example, the controller can solve the following equation:

$$\min_{\varepsilon m}\left[\left(\sum_m \int_0^{Lm} HSPRm + \varepsilon m \, dL\right) - WEPL, HRS\right] \equiv \min_{\varepsilon'm}\left[\sum_m WEPLm + \varepsilon'm - \right.$$

*WEPL, HRS*], where $\varepsilon m$, and $\varepsilon'm$ are the uncertainties associated to the HSPRm, and Lm.

Preferably, several **WEPL,HRS** are measured for several target spots **40si,j.**

The verification of the treatment plan can be performed during a treatment session by the controller **5.** The controller verifies if the water equivalent path length, **WEPL40s,0,** of a beam path comprised between the upstream boundary and the target spot **40s,** and being determined during the establishment of the treatment plan is equal or not to the actual value of the **WEPL40s,1** determined during the treatment session.

The treatment plan is then applied if WEPL40s,0 - $\delta \leq$ WEPL40s,1 $\leq$ WEPL40s,0 + $\delta$ with $\delta$ = 10 mm, preferably $\delta$ = 5 mm, more preferably $\delta$ = 3 mm.

If WEPL40s,1 $\geq$ WEPL40s,0 + $\delta$ or WEPL40s,1 $\leq$ WEPL40s,0 - $\delta$ with $\delta$ = 10 mm, preferably $\delta$ = 5 mm, more preferably $\delta$ = 3 mm, the controller can take further actions. For example, it can interrupt the treatment session and a new treatment plan can be established. Alternatively, the controller can modify the planned initial energy, E0 to a corrected initial energy, E1 yielding the Bragg peak at a treatment position, **BP1,** corresponding to the actual position, **P1,** of the target spot **40s** during the treatment session. The corrected treatment plan can then be delivered.

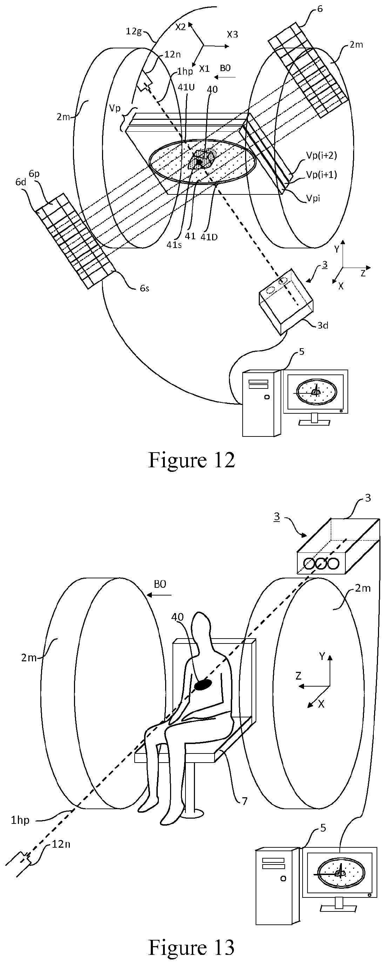

Figure 12 illustrates an example of the hadron therapy device according to the present invention further comprising a prompt-gamma detector or a PET scan. A prompt-gamma detector is a device for imaging prompt gammas emitted along the hadron beam path in the subject of interest. The hadrons of the hadron beam can enter in (non-elastic) collision with atomic nuclei of the tissues traversed. The collision can cause nuclear reaction that lead to the emission of gamma rays though desexcitation. These gamma rays are emitted very quickly (near immediately) after the passage of the hadrons. The gamma rays can be detected with detectors comprising, for example, a scintillator **6s,** a photomultiplier **6p** and a photon detector **6d.**

Alternatively, a positron emission tomography (PET) scan can be used. A PET scan is a device for imaging in 3D the concentration of $\beta^+$ (positron) emitter located along the hadron beam path in the subject of interest. A small fraction of the hadrons of the hadron beam create positron emitting isotopes (for example, $^{11}$C, $^{13}$N, $^{15}$O) through interactions with the atomic nuclei of the tissues traversed. These radio-active isotopes decay with emission of a positron which will annihilate with an electron leading to the emission of two gamma photons emitted in coincidence. The PET scan detects the source of emission of these two gamma photons and therefore measures the concentration of $\beta^+$ emitter. The concentration of $\beta^+$ emitter is related to the beam path of the hadron beam.

The prompt-gamma detector and the PET scan are additional detectors that can complement the HRS. They both rely on a treatment hadron beam. The emission of gamma photons is linked to the hadron beam and to the beam path. The emission occurs from the upstream boundary of the subject of interest to a position slightly upstream of the Bragg peak. These additional detectors offer additional information on the position of the Bragg peak and thus allow an improvement of the range determination of the hadron beam.

Figure 13 illustrates an example of the hadron therapy device according to the present invention further comprising a support for supporting a patient in a non-supine position. A low uncertainty on the position of the target tissue **40s** can permit large morphological differences between the establishment of the treatment plan and the treatment session. In this context, treating a patient in a non-supine position can be advantageous because it does not require a gantry. The beam nozzle is thus fixed and the cost of the apparatus is greatly decreased. The apparatus according to the embodiment of the Figure 13 can also comprise a prompt-gamma detector and/or a PET scan.

According to a second aspect, the present invention relates to methods for establishing and for validating a treatment plan.

As described above, a treatment plan can be established in a "classical" way by using a CT scan, for example, in following the steps of:

(A) running a CT scanner and / or a MRI;

(B) from the CT/MR images, performing a characterisation of the tissues within the portion of the subject of interest including a predicted beam path comprised between upstream boundary and target spot, this characterisation comprising an identification of the nature of the tissue m and of their thicknesses, **Lm;**

(C) an identification of a preliminary water equivalent path length **WEPL40s,0** of the beam path comprised between the upstream boundary **41U** and the target spot **40s;** and,

(D) computing a dose delivery scheme optimized with respect to the preliminary characterization, the computation including the computation of a planned initial energy, E0, of a treatment hadron beam, the computation being based on the value of **WEPL40s,0** and yielding the Bragg peak at a planned computed position, **BP0,** corresponding to the position of the target spot during the establishment of the treatment plan.

Alternatively, the treatment plan can be established in performing a MRI to obtain MR data of an imaging volume, **Vp,** comprising a target spot **40s.** An imaging hadron beam having an initial imaging beam energy, **E0p,** can be emitted along a beam path going through and beyond:

• the subject of interest extending along the beam path between upstream and downstream boundaries, and

• the target spot.

The MR data can be used to characterize the tissues **m** traversed by the hadron beam. The characterisation includes a determination of the nature of the tissue **m,** a measurement of the thicknesses **Lm** of the tissues **m,** and an estimation of the **HSPRm** of the tissues **m.**

The residual energy or, equivalently, the residual range of the imaging hadron beam is measured with a hadron radiography system (HRS). The difference between the initial energy **E0p** and the residual energy gives the absorbed energy that correspond to a water equivalent path length **WEPL,HRS** of said beam path between upstream and downstream boundaries.

From the **WEPL,HRS** and from the characterisation of the tissues **m,** the water equivalent path length **WEPLm** of tissues **m,** can be computed. Finally, the value of the **WEPL40s,0** of the beam path comprised between the upstream boundary and the target spot, is calculated using the **WEPLm's** of the tissues positioned upstream of and including the target spot. A planned initial energy E0 is then computed such that the position, **BP0,** of the Bragg peak of a treatment hadron beam position, corresponds to the position, **P0,** of the target spot **40s** during the treatment plan establishment. The computation is preferably done for several target spots **40si,j.**

The establishment of the treatment plan is performed before the treatment. The planned treatment may comprise a plurality of successive treatment session. Usually, the establishment of the treatment is performed several days to few weeks before the first treatment session.

A treatment session can include a step of validation of the planned energy, E0 before treating the patient. The validation is similar to the establishment of the treatment plan: an MRI is performed and an imaging hadron beam is emitted. The MR data are used to characterize the tissues traversed by the imaging hadron beam and a signal generated by the imaging hadron beam is used to compute the **WEPL,HRS.** From that, the **WEPLm** of tissues **m** are computed. The water equivalent path length **WEPL40s,1** of the beam path comprised between the upstream boundary and the target spot is computed based on the actual measurements of the day of the treatment session.

The actual value of the **WEPL40s,1** is then compared with the preliminary value of **WEPL40s,0** determined during the establishment of the treatment plan. If the WEPL40s,0 - $\delta \leq$ WEPL40s,1 $\leq$ WEPL40s,0 + $\delta$ with $\delta$ = 10 mm, preferably $\delta$ = 5 mm, more preferably $\delta$ = 3 mm, the planned initial energy, E0 is validated. If WEPL40s,1 $\geq$ WEPL40s,0 + $\delta$ or WEPL40s,1 $\leq$ WEPL40s,0 - $\delta$ with $\delta$ = 10 mm, preferably $\delta$ = 5 mm, more preferably $\delta$ = 3 mm, the treatment session can be stopped.

Alternatively, and preferably to the interruption of the treatment session, the planned initial energy, E0 can be modified to a corrected initial energy, E1, of a treatment hadron beam computed during the validation and based on the **WEPL40s,1,** said final energy, E1, yielding the Bragg peak at a treatment position, **BP1,** corresponding to the actual position of the target spot.

Preferably, the method described are performed with a medical apparatus according to the present invention. Preferably, the beam delivery system, the MRI and the HRS are located in the same room.

The present invention allows reducing range uncertainty of a hadron beam during a treatment session. The position and morphology of a target tissue **40** can evolve between a time, **t0,** of establishment of a treatment plan and a time, **t0** + $\Delta$**t3,** of a treatment session, being able to (partly) measure this evolution is crucial to perform a good treatment. A PT-MRI combined to a HRS can help to visualize the change of the morphologies and positions of the target tissue and surrounding tissues and to verify in situ the initial energy computed during the treatment plan. The apparatus according to the present invention can possibly allow correcting the initial energy to match the position of the target tissue and the position of the Bragg peak if the hadron beam. It thus reduces the risk of irradiating healthy tissue or missing target tissue that leads to the formation of new cancer and improves the efficiency of the treatment in allowing the adaptation of the dose delivered at a target spot.

**Claims**

1. A medical apparatus comprising:

   (A) a hadron therapy device (1) comprising a hadron source (10) adapted for directing an imaging hadron beam (1hp) having an initial imaging beam energy, EOp, along a beam path (Xp), said beam path going through and beyond:

      • a subject of interest comprising a plurality of tissues m and crossed by the beam path between upstream (41U) and downstream boundaries (41D), and
      • a target spot (40s) located inside a target tissue (40) of the subject of interest between the upstream and downstream boundaries;

   (B) a magnetic resonance imaging device (MRI) for acquiring magnetic resonance (MR) data within an imaging volume, Vp, comprising a portion of the subject of interest including the target spot and at least the portion of the beam path comprised between the upstream boundary and the target spot;

   **characterized in that,** the medical apparatus further comprises:

   (C) a hadron radiography system (HRS) adapted for acquiring a signal generated by the imaging hadron beam; and
   (D) a controller (5) configured for a determination of a water equivalent path length WEPL,HRS of the beam path between said upstream boundary and said downstream boundary, said determination being based, at least, on the signal acquired with the HRS.

2. The medical apparatus according to claim 1, wherein the controller (5) is further configured for computing the water equivalent path length, WEPLm, of the plurality tissues m, of thickness Lm, crossed by the beam path comprised between the upstream (41U) and downstream (41D) boundaries, the computation being based on the MR data and on the WEPL,HRS.

3. The medical apparatus according to claim 2, wherein the controller is further configured for performing, at a time t1 during a treatment session, a validation of a planned value of a water equivalent path length, WEPL40s,0, of a beam path comprised between the upstream (41U) boundary and the target spot (40s), wherein said planned value of the WEPL40s,0 had been determined previously at time t0 during a treatment session, said validation comprising:

   • a calculation of an actual value of a water equivalent path length WEPL40s,1 of a beam path (Xp) comprised between the upstream boundary and the target spot, said calculation being based on the WEPLm's of the plurality of tissues positioned upstream of and including the target tissue (40);
• a comparison of the actual value of the $WEPL_{40s,1}$ with the planned value of the $WEPL_{40s,0}$;
• if $WEPL_{40s,0} - \delta \leq WEPL_{40s,1} \leq WEPL_{40s,0} + \delta$ with $\delta$ = 10 mm, preferably $\delta$ = 5 mm, more preferably $\delta$ = 3 mm, then apply the treatment plan;
• if $WEPL_{40s,1} \geq WEPL_{40s,0} + \delta$ or $WEPL_{40s,1} \leq WEPL_{40s,0} - \delta$ with $\delta$ = 10 mm, preferably $\delta$ = 5 mm, more preferably $\delta$ = 3 mm, then take further actions.

4. The medical apparatus according to claim 3, wherein the further actions comprise a correction of:

   • a planned initial energy, E0, of a treatment hadron beam previously computed during a treatment plan and based on the planned value of the $WEPL_{40s,0}$, said planned initial energy, E0, yielding the Bragg peak at a planned position, BP0, corresponding to the position of the target spot as defined by treatment plan; to
   • a corrected initial energy, E1, of a treatment hadron beam computed during the validation and based on the actual value of the $WEPL_{40s,1}$, said corrected initial energy, E1, yielding the Bragg peak at a treatment position, BP1, corresponding to the actual position of the target spot.

5. The medical apparatus according to any one of the claims 2 to 4, wherein the controller is configured for establishing a treatment plan by:

   • calculating a preliminary value of $WEPL_{40s,0}$ of the target spot said calculation being based on the WEPLm's; and
   • computing a planned initial energy, E0, of a treatment hadron beam, the computation being based on the value of $WEPL_{40s,0}$ and yielding the Bragg peak BP0, at a planned position corresponding to the position of the target spot during the establishment of the treatment plan.

6. The medical apparatus according to any one of the previous claims, wherein the hadron radiography system comprises one or more of the following detectors: a range telescope (3t), a calorimeter (3c), or a spectrometer (3s).

7. The medical apparatus according to any one of the previous claims, further comprising at least one of a prompt-gamma detector (6) and a PET scan (6).

8. The medical apparatus according to any one of the previous claims, comprising a support (7) for supporting a patient in a non-supine position.

9. A method for validating a planned initial energy, E0, of a treatment hadron beam computed during a treatment plan for irradiation of a target spot (40s) inside a subject of interest, said subject of interest comprising a plurality of tissues m, said method comprising the following steps:

   (A) performing a magnetic resonance (MR) imaging of an imaging volume, Vp, comprising the target spot, and acquiring MR data;
   (B) emitting, along a beam path (Xp), an imaging hadron beam (1hp) having an initial imaging beam energy, EOp, said beam path going through and beyond:

   • the subject of interest extending along the beam path between upstream (41U) and downstream (41 D) boundaries, and
   • the target spot;

   **characterized in that** said method comprises the following additional steps:

   (C) detecting a signal generated by said imaging hadron beam with a hadron radiography system (HRS);
   (D) determining, from said signal, the water equivalent path length WEPL,HRS of said beam path between upstream and downstream boundaries;
   (E) computing the WEPLm of tissues m, of thickness Lm, crossed by the beam path comprised between the upstream and downstream boundaries, the computation being based on the MR data and on the WEPL,HRS.
   (F) computing the value of an actual value of the $WEPL_{40s,1}$ of the beam path comprised between the upstream boundary and the target spot, said calculation being based on the WEPLm's of the tissues positioned upstream of and including the target spot;
   (G) comparing the actual value of the $WEPL_{40s,1}$ with a preliminary value of $WEPL_{40s,0}$ determined during

the establishment of the treatment plan;

(H) if WEPL40s,0 - tol ≤ WEPL40s,1 ≤ WEPL40s,0 + tol with tol = 10 mm, preferably tol = 5 mm, more preferably tol = 3 mm, validating the planned initial energy, E0;

(I) if WEPL40s,1 ≥ WEPL40s,0 + tol or WEPL40s,1 ≤ WEPL40s,0 - tol with tol = 10 mm, preferably tol = 5 mm, more preferably tol = 3 mm, taking further actions.

10. The method according to claim 9, wherein the further actions comprise a modification of the value of the planned initial energy, E0 to a value of a corrected initial energy, E1, of a treatment hadron beam computed during the validation and based on the WEPL40s,1, said corrected initial energy, E1, yielding the Bragg peak at a treatment position, BP1, corresponding to the actual position of the target spot.

11. The method according to claim 9 or 10, wherein the validation of the treatment plan is done at a time t0 + Δti and is preceded by an establishment of the treatment plan at a time t0, said establishment comprising the following steps:

(A) running a CT scanner and / or a magnetic resonance imaging to yield a preliminary characterisation of the tissues within the portion of the subject of interest including a predicted beam path comprised between upstream boundary and target spot, and an identification of a preliminary WEPL40s,0 of the beam path comprised between the upstream boundary and the target spot (40s); and,

(B) computing a dose delivery scheme optimized with respect to the preliminary characterization, the computation including the computation of a planned initial energy, E0, of a treatment hadron beam, the computation being based on the value of WEPL40s,0 and yielding the Bragg peak at a planned position, BP0, corresponding to the position of the target spot during the establishment of the treatment plan.

12. A method of establishment of a treatment plan of a treatment hadron beam for irradiation of a target spot (40s) inside a subject of interest, said method comprising the following steps:

(A)performing a magnetic resonance (MR) imaging of an imaging volume, Vp, comprising a target spot for acquiring MR data;

(B) emitting, along a beam path, an imaging hadron beam having an initial imaging beam energy, EOp, said beam path going through and beyond:

• the subject of interest extending along the beam path between upstream and downstream boundaries, and
• the target spot (40s);

**characterized in that** said method comprises the following additional steps:

(C) detecting a signal generated by said imaging hadron beam with a hadron radiography system (HRS);

(D) determining, from said signal, the water equivalent path length WEPL,HRS of said beam path between upstream and downstream boundaries;

(E) computing the WEPLm's of tissues m, of thickness Lm, crossed by the beam path comprised between the upstream and downstream boundaries, the computation being based on the MR data and on the WEPL,HRS.

(F) computing of a value of the WEPL40s,0 of the beam path comprised between the upstream boundary and the target spot, said calculation being based on the WEPLm's of the tissues positioned upstream of and including the target spot;

(G) computing a dose delivery scheme including the computation of a planned initial energy, E0, of a treatment hadron beam, the computation being based on the value of WEPL40s,0 and yielding the Bragg peak at a planned position, BP0, corresponding to the position of the target spot during the establishment of the treatment plan.

13. The method according to claim 12, further comprising: a preliminary characterisation, of the tissues crossed by the beam path within the portion of the subject of interest including the beam path comprised between upstream and downstream boundaries, said characterisation being based on the MR data.

14. The method according to any one of the claims 9 to 11, wherein said validation of the treatment plan is done at a time t1 and is preceded by an establishment of the treatment plan at a time t0, said establishment being done according to the claim 12 or 13.

15. The method according to any one of the claims 10 to 14, further comprising the step of providing a medical apparatus according to any one of the claims 1 to 9.

Figure 1 (Prior art)

Figure 2

(a)

(b)

Figure 3

Figure 4

Figure 5

Figure 6

Figure 7

32t 32t 32t 32t 32t 32t 32t 32t 32t 32t

3

31t

1hp

33t

3t

E-loss (MeV)

Xh (mm) or WEPL (mm)

Figure 8(a)

1hp

31c

3

3c

33c

32c

Figure 8(b)

Figure 8(c)

Figure 9

Figure 10

Figure 11

Figure 12

Figure 13

Europäisches
Patentamt

European
Patent Office

Office européen
des brevets

## EUROPEAN SEARCH REPORT

Application Number

EP 16 19 2790

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 853 292 A1 (ION BEAM APPLIC SA [BE]) 1 April 2015 (2015-04-01) * abstract * * paragraph [0069] * ----- | 1-15 | INV. A61N5/10 A61B6/03 A61B6/00 A61B5/055 |
| A | US 2015/217139 A1 (BERT CHRISTOPH [DE] ET AL) 6 August 2015 (2015-08-06) * abstract * * paragraph [0043] - paragraph [0048] * ----- | 1-15 | |
| A | Dirk Boye ET AL: "Mapping motion from 4D-MRI to 3D-CT for use in 4D dose calculations: A technical feasibility study", Medical Physics, 7 May 2013 (2013-05-07), page 61702, XP055344955, DOI: 10.1118/1.4801914 Retrieved from the Internet: URL:http://onlinelibrary.wiley.com/doi/10.1118/1.4801914/pdf [retrieved on 2017-02-13] * abstract; figure 1 * ----- | 1-15 | TECHNICAL FIELDS SEARCHED (IPC) A61N A61B |

The present search report has been drawn up for all claims

| Place of search | Date of completion of the search | Examiner |
|---|---|---|
| Munich | 13 February 2017 | Kajzar, Anna |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

  .................................................................
& : member of the same patent family, corresponding
document

EPO FORM 1503 03.82 (P04C01)

**EP 3 305 367 A1**

## ANNEX TO THE EUROPEAN SEARCH REPORT
## ON EUROPEAN PATENT APPLICATION NO.

EP 16 19 2790

This annex lists the patent family members relating to the patent documents cited in the above-mentioned European search report.
The members are as contained in the European Patent Office EDP file on
The European Patent Office is in no way liable for these particulars which are merely given for the purpose of information.

13-02-2017

| Patent document cited in search report | | Publication date | Patent family member(s) | Publication date |
|---|---|---|---|---|
| EP 2853292 | A1 | 01-04-2015 | EP 2853292 A1<br>JP 2015066449 A<br>US 2015094517 A1 | 01-04-2015<br>13-04-2015<br>02-04-2015 |
| US 2015217139 | A1 | 06-08-2015 | CN 104540547 A<br>DE 102012004170 A1<br>EP 2822651 A1<br>JP 2015510781 A<br>US 2015217139 A1<br>WO 2013131890 A1 | 22-04-2015<br>05-09-2013<br>14-01-2015<br>13-04-2015<br>06-08-2015<br>12-09-2013 |

EPO FORM P0459

For more details about this annex : see Official Journal of the European Patent Office, No. 12/82

**EP 3 305 367 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8427148 B
- US 4870287 A

- EP 0186238 A